# EUROPÄISCHE PATENTSCHRIFT

(11) **EP 0 975 298 B1**
(45) Veröffentlichungstag und Bekanntmachung des Hinweises auf die Patenterteilung: **24.07.2002**
(21) Anmeldenummer: 98919281.0
(22) Anmeldetag: 17.04.1998
(51) Int. Cl.: A61F 13/15

(54) **ATMUNGSAKTIVE WINDEL**
BREATHABLE DIAPER
COUCHE IMPER-RESPIRANTE

(30) Priorität: 18.04.1997 DE 19716253
(43) Veröffentlichungstag der Anmeldung: 02.02.2000
(73) Patentinhaber: Paul Hartmann Aktiengesellschaft, 89522 Heidenheim (DE)
(72) Erfinder: MALOWANIEC, Krzysztof, D., D-89522 Heidenheim (DE); OLTMANN, Eckard, D-89520 Heidenheim (DE)
(74) Vertreter: Friz, Oliver
(86) Internationale Anmeldenummer: EP9802279
(87) Internationale Veröffentlichungsnummer: WO9847453

(56) Entgegenhaltungen:
- EP-A- 0 386 815
- EP-A- 0 412 549
- EP-A- 0 446 867
- WO-A-96/05792
- WO-A-96/31176
- WO-A-96/31178
- DE-A- 3 245 196
- GB-A- 2 268 389

## Beschreibung

Die Erfindung betrifft eine Wegwerfwindel mit den Merkmalen des Oberbegriffs des Anspruchs 1 wie sie aus der US-PS 5,571,096 bekannt ist.

Es sind ferner Wegwerfwindeln bekannt, die neben einem flüssigkeitsdurchlässigen Deckblatt und einem die Flüssigkeit absorbierenden Saugkörper ein flüssigkeitsundurchlässiges Rückenblatt aufweisen. Das flüssigkeitsundurchlässige Rückenblatt hält die vom Saugkörper absorbierten Körperflüssigkeiten zurück und verhindert den Durchgang der Körperflüssigkeiten durch die Windel hindurch, fungiert also als Wäscheschutz.

Üblicherweise werden dünne Kunststoffolien als flüssigkeitsundurchlässiges Rückenblatt eingesetzt, die nicht nur flüssigkeits- sondern auch dampfdicht sind.

Das vollständige Zurückhalten der Körperflüssigkeiten in der Windel verursacht jedoch einen hautphysiologisch und vom Träger der Windel auch subjektiv so empfundenen unvorteilhaften Zustand (Nässegefühl, warmer Urin, Hautreizungen, Brennen der Haut etc.). Zudem verhindert ein flüssigkeits- und dampfdichtes Rückenblatt eine zumindest teilweise mögliche Regeneration des Absorptionsvermögens des Saugkörpers.

Neben dem Erfordernis, das Rückenblatt wasserdampfdurchlässig aber flüssigkeitsdicht auszugestalten, um das subjektive Empfinden des nassen Saugkörpers und die hautphysiologischen Folgeerscheinungen zu minimieren, existiert ein weiteres Problem. Dieses betrifft ebenfalls das Mikroklima im Bereich der an den Körper angelegten Windel, es handelt sich hierbei aber um ein von dem erstgenannten unabhängiges Problem:

Bei üblicherweise sanduhrförmiger Gestaltung einer Wegwerfwindel, insbesondere dann, wenn der Saugkörper lediglich auf einen schmalen, bevorzugt rechteckigen, im Zentralbereich der Windel angeordneten Streifen begrenzt ist, bestehen relativ breite Seitenteile der Windel lediglich aus der körperseitigen Abdeckung und dem Rückenblatt. Diese Seitenteile stehen - teilweise überlappend - größflächig in direktem Kontakt mit der Haut des Trägers der Windel. Bei instationären Tragebedingungen kommt es nun beim Menschen infolge erhöhter wie auch zeitlich variierender physischer Belastung häufig zur impulsartigen Abgabe größerer Wasserdampfmengen oder sogar flüssigen Schweißes.

Es ist somit Aufgabe der Erfindung, eine Windel zu konstruieren, bei der ein erwünschter Durchtritt von Wasserdampf aber auch eine 100%-ige Rückhaltefähigkeit für Urin gewährleistet ist und die im Bereich der Seitenteile ein angenehmes Mikroklima und damit einen hohen Tragkomfort vermittelt.

Das Rückenblatt soll gleichwohl mit einer ausreichend hohen Festigkeit ausgestattet sein. Zudem soll eine wirtschaftliche Herstellbarkeit gewährleistet sein, was insbesondere bedeutet, einen mehrschichtigen Aufbau des Rückenblattes zu vermeiden.

Diese Aufgabe wird gelöst durch eine Windel mit den Merkmalen des Anspruches 1.

Mit der Erfindung wird also vorgeschlagen, durch geeignete Mittel zu verhindern, dass Flüssigkeit bzw. Urin in die Seitenteile voranschreitet, und im Bereich eines bestimmungsgemäßen Flüssigkeitsspeichers in Form des Saugkörpers einen gegenüber den Seitenteilen höheren Dampfaustausch vorzusehen. Hiermit wird erfindungsgemäß erreicht, dass der Flüssigkeitsspeicher zumindest teilweise regenerierbar ist, indem ein Teil der Flüssigkeit in Form von Dampf austreten kann, ohne dass ein weiterer Kühleffekt, der im Bereich des Seitenteils als unangenehm empfunden werden kann, wirksam ist.

Es wird ausdrücklich darauf hingewiesen, dass vom vorstehend erläuterten Erfindungsgedanken nicht nur Wegwerfwindeln, sondern auch andere Hygieneprodukte erfasst sind.

Weitere Merkmale, Einzelheiten und Vorteile der Erfindung ergeben sich aus den Patentansprüchen sowie der zeichnerischen Darstellung und nachfolgenden Beschreibung einer bevorzugten Ausführungsform der erfindungsgemäßen Windel. In der Zeichnung zeigt:
- Figur 1: eine Draufsicht auf eine erfindungsgemäße Windel; und
- Figur 2: eine Schnittansicht gesehen in Richtung der Pfeile II-II mit Einteilungen verschiedener Bereiche der Windel.

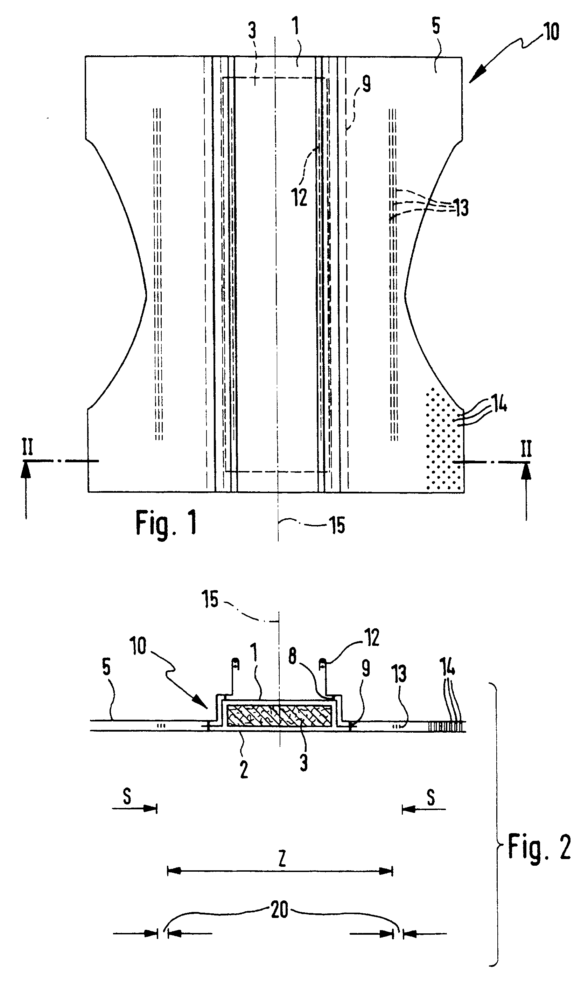

Die Figuren 1 und 2 zeigen eine erfindungsgemäße Wegwerfwindel. Die Wegwerfwindel 10 besitzt eine flüssigkeitsdurchlässige Abdeckung 1. Die flüssigkeitsdurchlässige Abdeckung ist bevorzugt ein Vliesstoff, kann aber auch eine mit Löchern versehene Folie sein. Sie ist im einfachsten Fall einschichtig ausgeführt, zwei- oder mehrschichtige Ausführungen sind ebenfalls denkbar. Zwischen der flüssigkeitsdurchlässigen Abdeckung 1 und dem Rückenblatt 2 ist der Saugkörper 3 angeordnet. Der Saugkörper 3 besteht beispielsweise aus Zellstofflocken und superabsorbierenden Stoffen, kann aber auch synthetische Fasern enthalten. Der Saugkörper kann außerdem ein- oder mehrschichtig ausgebildet sein. Die Form des Saugkörpers ist bevorzugt näherungsweise rechteckig, sie kann auch in bekannter hier nicht dargestellter Weise sanduhrförmig oder T-förmig sein. Die Breite der flüssigkeitsdurchlässigen Abdeckung 1 ist geringer als die Breite des Rückenblattes 2 aber größer als die des Saugkörpers 3 an seiner schmalsten Stelle.

Zwei Streifen hydrophober Dichtklappen 5 schließen mit ihrer Außenkante jeweils bündig mit der Außenkante des Rückenblattes 2 ab und erstrecken sich mit ihrer Innenkante über die Außenkante der Abdeckung 1 und über den Saugkörperrand hinweg. Das Dichtklappenmaterial ist bevorzugt ein Vliesstoff, der einschichtig, aber z. Bsp. in Form des bekannten SM- (Spunbond-Meltblown) oder SMS(Spunbond-Meltblown-Spunbond) Aufbaus aber auch mehrschichtig sein kann.

Die Dichtklappen 5 sind mit der flüssigkeitsdurchlässigen Abdeckung 1 entlang einer der Longitudinalachse 15 der Windel parallelen Linie 8 etwa im Bereich der Seitenränder des Saugkörpers 3 verbunden, z. Bsp. durch Kleben, Siegeln, Verschweißen o.ä. Diese Linie bildet den Sockel der inneren Beinabschlüsse. An den Innenkanten der Dichtklappen 5 sind Elastifizierungsmittel 12 befestigt. Wie in der Zeichnung veranschaulicht können die Elastifizierungsmittel aus mehreren Elastifizierungselementen, z.Bsp. jeweils zwei elastischen Fäden bestehen.

Zumindest im Schrittbereich sind die flüssigkeitsdurchlässige Abdeckung 1 und die Dichtklappen 5 im Abschnitt zwischen der beschriebenen Sockellinie 8 und der Innenkante der Dichtklappen nicht miteinander verbunden. Dadurch werden die Dichtklappen im Schrittbereich - verursacht durch das elastische Material - gezwungen aufzustehen, womit die inneren Beinabschlüsse gebildet werden, die eine erste mechanische Sperre gegen das weitere laterale Voranschreiten der Körperflüssigkeiten bilden. Außerhalb der Sockellinie 8 sind Dichtklappen und Rückenblatt ebenfalls entlang einer der Longitudinalachse 15 parallelen Linie 9 flüssigkeitsdicht z. Bsp. durch Kleben, Siegeln, Verschweißen o.ä. miteinander verbunden.

Falls die Außenkante der flüssigkeitsdurchlässigen Abdeckung 1 wie in der Zeichnung dargestellt über diese Linie 9 hinausreicht ist, wird die Abdeckung durch die Verbindung mit durchdrungen. Mit dieser Linie 9 ist eine zweite mechanische Sperre errichtet, die ein seitliches Voranschreiten der absorbierten Körperflüssigkeiten innerhalb der Windel blockiert.

Außerhalb der Linie 9 und auch außerhalb der Außenkante der flüssigkeitsdurchlässigen Abdeckung 1 sind zwischen Dichtklappen 5 und Rückenblatt 2 sich longitudinal erstreckende Elastifizierungsmittel 13 angebracht, womit die äußeren Beinabschlüsse gebildet werden. Sollte Flüssigkeit die beiden erstgenannten Sperren überwunden haben, so bildet der durch Kleben, Schweißen, Siegeln o.ä. vollzogene dichte Verbund zwischen Rückenblatt, Dichtklappen und Elastifizierungsmittel eine letzte Barriere vor dem weiteren Voranschreiten von Flüssigkeit in die - wie unten noch erläutert wird - flüssigkeitsundichten Seitenteile der Windel. Diese Barriere bildet gleichzeitig die Grenze 20 zwischen Zentralbereich (Z) und Seitenteilen (S) der Windel. Setzen sich die Elastifizierungsmittel aus mehreren Elastifizierungselementen zusammen, z. Bsp. wie in der Zeichnung dargestellt aus drei Fäden elastischen Materials so besitzt die Grenze 20 eine flächige Ausdehnung. Unter "außerhalb der Grenze 20" wird dann der Bereich außerhalb des äußersten elastischen Fadens verstanden. Ebenso bedeutet "innerhalb der Grenzen 20" und "zwischen den Grenzen 20", daß der Bereich zwischen den innersten elastischen Fäden bzw. innerhalb derselben gemeint ist.

Das Rückenblatt 2 ist im Zentralbereich (Z) innerhalb der Grenzen 20 wasserdampfdurchlässig. Bei Betrachtung des Rückenblattes innerhalb der Grenzen 20 verstehen wir unter der Eigenschaft "wasserdampfdurchlässig" eine Wert von mehr als 1000 g/m2 über einen Zeitraum von 24 Stunden, ermittelt nach DIN 53122, Blatt 1.

Die Wasserdampfdurchlässigkeit innerhalb der Grenzen 20 erhöht den Tragekomfort der Windel, da sich in diesem Bereich der mit Körperflüssigkeiten je nach Nutzungsintensität mehr oder weniger gesättigte Saugkörper befindet. Das Diffundieren von Wasserdampf durch das Rückenblatt hindurch ermöglicht eine zumindest teilweise Trocknung des Saugkörpers. Damit reduziert sich das unangenehme Nässegefühl des Trägers und das Ausmaß an Hautreizungen. Die Verdunstungskälte verschafft dem Windelträger zudem eine als positiv empfundene Kühlung.

Trotz der hohen Wasserdampfdurchlässigkeit ist das Rückenblatt flüssigkeitsdicht, das heißt unter den Bedingungen des Gebrauchs der Windel sollte kein Wasser in flüssiger Form das Rückenblatt durchdringen können. Unter flüssigkeitsdicht im Sinne der vorliegenden Erfindung verstehen wir eine Wassersäule von mindestens 250 mm, ermittelt nach der in DIN EN 20811 beschriebenen Prüfmethode.

Flächenbildende, wasserdampfdurchlässige aber flüssigkeitsdichte Materialien und Verfahren zu deren Herstellung sind bekannt (z. Bsp. DE PS 3121040, DE OS 3306843, G. Pinchard (presentation "Breathable Films" at "Absorbent Products Conference", Oct. 16 and 17, 1996 in San Antonio, Texas, USA), 1996, EP 0259003). Grundsätzlich besteht die Möglichkeit, das Rückenblattmaterial entweder - was durch unterschiedliche Methoden erreicht werden kann - mit Mikroporen auszustatten, um Wasserdampf die Möglichkeit zu geben, auf mechanischem Wege zu penetrieren oder Materialien zu verwenden, die Wasserdampf mithilfe der Chemiesorption penetrieren lassen, wie es beispielsweise bei Zellglasfolien seit langem bekannt ist.

Bei Verwendung eines mikroporösen Rückenblattmaterials, weisen die Poren - bei hinsichtlich ihrer Geometrie idealisiert runder Betrachtung der Poren - bevorzugt einen durchschnittlichen Durchmesser von 0,2-10 µm auf. Selbstverständlich können die Poren auch andere geometrische Formen annehmen.

Das Rückenblatt ist gemäß einer bevorzugten Ausführungsform einschichtig. Bei einer besonders bevorzugten Ausführungsform der Erfindung (Anspruch 14) ist das Rückenblatt ein einschichtiger Vlies/Folien-Verbund. Die Folienkomponente dringt dabei in die dreidimensionale Vliesstruktur ein, wodurch makroskopisch, das heißt mit bloßem Auge nicht mehr zwischen zwei Schichten unterschieden werden kann. Damit wird dem Rückenblattmaterial ein textiler, da faseriger Eindruck vermittelt. Trotz des den mikroporösen Folien anhaftenden Festigkeitsproblems können das Flächengewicht von Folie- und Vlieskomponente und damit auch des Verbundes aufgrund dieses Aufbaus sehr niedrig gewählt werden, da eine Art faserverstärkter Verbundwerkstoff entsteht. Damit sind wirtschaftliche Vorteile verbunden, wie auch ein höherer Tragekomfort, da ein dünnes Rückenblatt nur geringfügig zum ungewünschten Auftragen der Windel beiträgt.

Außerhalb der Grenzen 20 weist das Rückenblatt 2 Makroporen 14 auf. Unter Makroporen werden alle Art von Öffnungen verstanden, unabhängig von deren Geometrie und unabhängig von der Art und dem Zeitpunkt des Einbringens der Öffnungen. Die einzelnen Makroporen weisen in einer bevorzugten Ausführung der Erfindung eine Projektionsfläche von mindestens 0,1 mm² höchstens aber 5,0 mm² auf. Die Summe der offenen Flächen aller Makroporen sollte bezogen auf die Gesamtfläche der Seitenteile mindestens 3% betragen. Diese Größenordnung ermöglicht, daß ein Luftaustausch zwischen der Haut des Trägers und der Außenseite der Windel stattfinden kann und somit Wasserdampf sehr effektiv auf konvektivem Weg transportiert werden kann. Eine zu hohe Luftdurchlässigkeit kann allerdings zu einem unangenehm starken Kühleffekt führen, weshalb es sinnvoll ist, die Luftdurchlässigkeit über die Anzahl und Größe der Makroporen zu beschränken. Der Anteil der offenen Fläche sollte daher nicht mehr als 25% betragen.

Im Falle, daß das Material der Dichtklappen 5 die Luftdurchlässigkeit der Seitenteile der Windel zu stark behindert, werden die Dichtklappen gemäß einer weiteren Ausführungsform der Erfindung ebenfalls mit Makroporen versehen.

Da die durch die Makroporen hervorgerufene Luftdurchlässigkeit auch ein Durchdringen von Flüssigkeit erlaubt, ist es besonders bedeutsam, die oben beschriebenen drei Flüssigkeitssperren auszuführen, um ein Vordringen von absorbierter Körperflüssigkeit jenseits der Grenzen 20 sicher zu vermeiden.

Anders ausgedrückt erlaubt es die dreifache Flüssigkeitssperre besonders gut, die Windel den Erfordernissen im Tragezustand hinsichtlich der sich bildenden unterschiedlichen Mikroklimata im Zentralbereich und in den Seitenteilen anzupassen. Dies hat auch wirtschaftliche Vorteile, da der Aufwand des Bereitstellens eines wasserdampfdurchlässigen aber flüssigkeitsdichten Rückenblattes auf den Zentralbereich der Windel beschränkt werden kann. Gemäß Anspruch 2 ist die Wasserdampfdurchlässigkeit innerhalb der Grenzen 20 deshalb bevorzugt höher als außerhalb der Grenzen 20. Ein weiterer Vorteil dieser Ausführungsform der Erfindung ist, daß im Bereich der Seitenteile über den Luft- und damit auch Wasserdampftransport durch die Makroporen hinaus kein weiterer Kühleffekt wirksam ist, der wie oben erläutert im Bereich der Seitenteile als unangenehm empfunden werden kann. Durch die Wahl der Anzahl an Poren und deren Größe läßt sich die Wasserdampfdurchlässigkeit außerhalb der Grenzen 20 auf einfache Weise regeln.

Das Rückenblatt kann einstückig sein, es kann aber auch mehrteilig ausgeführt sein, derart, daß ein innerhalb der Grenzen 20 angeordneter wasserdampfdurchlässiger aber flüssigkeitsdichter Mittelstreifen beidseitig entlang den Grenzen 20 mit je einem luftdurchlässigen Streifen durch Schweißen, Siegeln, Kleben o.ä. verbunden ist.

## Patentansprüche

1. Wegwerfwindel, umfassend eine flüssigkeitsdurchlässige Abdeckung (1), ein Rückenblatt (2), einen in einem Zentralbereich (Z) der Windel zwischen der flüssigkeitsdurchlässigen Abdeckung (1) und dem Rückenblatt (2) befindlichen Saugkörper (3), ein Paar hydrophobe Dichtklappen (5), deren Innenkante sich über einen seitlichen Rand des Saugkörpers (3) nach innen hinwegerstreckt, wobei die Dichtklappen (5) außerhalb des Saugkörpers (3) entlang einer jeweiligen zur Längsachse (15) der Windel parallelen Linie (9) angefügt sind und wobei sich parallel zur Längsachse (15) der Windel erstreckende Elastifizierungsmittel vorgesehen sind, von denen ein erstes Paar (13) im Bereich außerhalb der jeweiligen Linie (9) zwischen den Dichtklappen (5) und dem Rückenblatt (2) fixiert ist und so ein erstes Paar äußerer Beinabschlüsse bildet und ein zweites Paar (12) von Elastifizierungsmitteln im Bereich zwischen den jeweiligen Linien (9) an den Dichtklappen (5) im Bereich von deren Innenkante fixiert ist, um so ein zweites Paar innerer Beinabschlüsse zu bilden, wobei das Rückenblatt (2) im Zentralbereich (Z) zwischen den Grenzen (20), die durch das äußere Paar der Elastifizierungsmittel (13) gebildet werden, wasserdampfdurchlässig aber flüssigkeitsdicht und in Seitenteilen (S) außerhalb des Zentralbereichs (Z) und der Grenzen (20) luftdurchlässig und wasserdampfdurchlässig ist, **dadurch gekennzeichnet, daß** die Dichtklappen (5) entlang der jeweiligen Linie (9) abdichtend mit dem Rückenblatt (2) verbunden sind und je eine Flüssigkeitsbarriere bilden, welche der Flüssigkeit den Weg zwischen Rückenblatt (2) und Dichtklappen (5) in die auch luftdurchlässigen Seitenteile (S) versperrt, und daß die Wasserdampfdurchlässigkeit des Rückenblattes (2) im Zentralbereich (Z) zwischen den Grenzen (20) größer ist als in den Seitenteilen (S) außerhalb dieser Grenzen (20) ist.

2. Wegwerfwindel nach Anspruch 1, **dadurch gekennzeichnet, daß** die äußeren Elastifizierungsmittel (13) bzw. die dadurch gebildeten Beinabschlüsse so zwischen den Dichtklappen (5) und dem Rückenblatt (2) angeordnet sind, dass sie eine weitere Flüssigkeitsbarriere bilden, welche ein laterales Voranschreiten der Flüssigkeit in Richtung auf die Seitenteile (S) verhindert.

3. Wegwerfwindel nach Anspruch 1 oder 2, **dadurch gekennzeichnet, daß** das Rückenblatt (2) so ausgebildet ist, dass es Wasserdampf durch den Vorgang der Chemisorption durchläßt.

4. Wegwerfwindel nach Anspruch 3, **dadurch gekennzeichnet, daß** die Eigenschaft des Rückenblattes (2), Wasserdampf durch den Vorgang der Chemisorption durchzulassen, auf den Zentralbereich (Z) zwischen den Grenzen (20) beschränkt ist.

5. Wegwerfwindel nach einem der vorherigen Ansprüche, **dadurch gekennzeichnet, daß** das Rückenblatt (2) Mikroporen zum Durchlassen von Wasserdampf aufweist.

6. Wegwerfwindel nach Anspruch 5, **dadurch gekennzeichnet, daß** die Mikroporen auf den Zentralbereich (Z) zwischen den Grenzen (20) beschränkt sind.

7. Wegwerfwindel nach Anspruch 5 oder 6, **dadurch gekennzeichnet, daß** die Mikroporen einen Durchmesser von 0,2-10 µm aufweisen.

8. Wegwerfwindel nach einem der vorherigen Ansprüche, **dadurch gekennzeichnet, daß** das Rückenblatt (2) außerhalb des durch die Grenzen (20) begrenzten Zentralbereichs (Z) Makroporen aufweist.

9. Wegwerfwindel nach Anspruch 8, **dadurch gekennzeichnet, daß** die Größe der Projektionsfläche der Makroporen 0,10-5,0 mm² beträgt.

10. Wegwerfwindel nach Anspruch 8 oder 9, **dadurch gekennzeichnet, daß** die Dichtklappen (5) außerhalb des durch die Grenzen (20) begrenzten Zentralbereichs (Z) Makroporen aufweisen.

11. Wegwerfwindel nach Anspruch 10, **dadurch gekennzeichnet, daß** die Makroporen der Dichtklappen (5) eine Projektionsfläche zwischen 0,10 und 5,0 mm² aufweisen.

12. Wegwerfwindel nach einem der vorherigen Ansprüche, **dadurch gekennzeichnet, daß** das Rückenblatt (2) eine einschichtige Struktur aufweist.

13. Wegwerfwindel nach einem der vorherigen Ansprüche, **dadurch gekennzeichnet, daß** das Rückenblatt (2) wenigstens abschnittsweise einen Vlies/Folien-Verbund umfaßt, derart daß die Folienkomponente in die dreidimensionale Struktur der Vlieskomponente eindringt.

## Claims

1. Disposable nappy comprising a liquid-permeable covering (1), a backing sheet (2), an absorbent member (3) located in a central region (Z) of the nappy between the liquid-permeable covering (1) and the backing sheet (2), a pair hydrophobic sealing flaps (5) of which the internal edge extends inwardly beyond a lateral border of the absorbent member (3), wherein the sealing flaps (5) are attached outside the absorbent member (3) along a respective line (9) parallel to the longitudinal axis (15) of the nappy and wherein elasticising means extending parallel to the longitudinal axis (15) of the nappy are provided, a first pair (13) of elasticising means being fixed in the region outside the respective line (9) between the sealing flaps and the backing sheet (2) and thus forming a first pair of external leg seals and a second pair (12) of elasticising means being fixed in the region between the respective lines (9) to the sealing flaps (5) in the internal edge region thereof and thus forming a second pair of inner leg seals, wherein the backing sheet (2) is water vapour-permeable but liquid-tight in the central region (Z) between the limits (20) formed by the outer pair of elasticising means (13) and is air-permeable and water vapour-permeable in lateral parts (S) outside the central region (Z) and the limits (20), **characterised in that** the sealing flaps (5) are connected in a sealing manner to the backing sheet (2) along the respective line (9) and form a respective liquid barrier which blocks the path of the liquid between the backing sheet (2) and sealing flaps (5) into the lateral parts (S) which are also air-permeable and **in that** the water vapour permeability of the backing sheet (2) is greater in the central region (Z) between the limits (20) than in the lateral parts (S) outside these limits (20).

2. Disposable nappy according to claim 1, **characterised in that** the outer elasticising means (13) or the leg seals formed thereby are arranged between the sealing flaps (5) and the backing sheet (2) in such a way that they form a further liquid barrier which prevents lateral progress of the liquid toward the lateral parts (S).

3. Disposable nappy according to claim 1 or 2, **characterised in that** the backing sheet (2) is constructed so as to be permeable to water vapour by chemical absorption.

4. Disposable nappy according to claim 3, **characterised in that** the property of the backing sheet (2) of being permeable to water vapour by chemical absorption is restricted to the central region (Z) between the limits (20).

5. Disposable nappy according to any of the preceding claims, **characterised in that** the backing sheet (2) comprises macropores for the permeation of water vapour.

6. Disposable nappy according to claim 5, **characterised in that** the macropores are restricted to the central region (Z) between the limits (20).

7. Disposable nappy according to claim 5 or 6, **characterised in that** the macropores have a diameter of 0.2 to 10 µm.

8. Disposable nappy according to any of the preceding claims, **characterised in that** the backing sheet (2) has macropores outside the central region (Z) limited by the limits (20).

9. Disposable nappy according to claim 8, **characterised in that** the size of the projected area of the macropores is 0.10 to 5.0 mm².

10. Disposable nappy according to claim 8 or 9, **characterised in that** the sealing flaps (5) comprise macropores outside the central region (Z) limited by the limits (20).

11. Disposable nappy according to claim 10, **characterised in that** the macropores of the sealing flaps (5) have a projected area of between 0.10 and 5.0 mm².

12. Disposable nappy according to any of the preceding claims, **characterised in that** the backing sheet (2) has a single layered structure.

13. Disposable nappy according to any of the preceding claims, **characterised in that** the backing sheet (2) comprises a non-woven/film composite at least in certain regions, in such a way that the film component penetrates into the three-dimensional structure of the non-woven component.

## Revendications

1. Couche jetable comprenant une couverture perméable aux liquides (1), une feuille arrière (2), un corps d'absorption (3) situé dans une zone centrale (Z) de la couche entre la couverture perméable aux liquides (1) et la feuille arrière (2), une paire de rabats étanches (5) hydrophobes, dont le bord intérieur s'étend par-dessus un bord latéral du corps d'absorption (3) vers l'intérieur, les rabats étanches (5) étant raccordés à l'extérieur du corps d'absorption (3) à chaque fois le long d'une ligne (9) parallèle à l'axe longitudinal (15) de la couche et des moyens d'élastification s'étendant parallèlement à l'axe longitudinal (15) de la couche étant prévus, dont une première paire (13) est localisée dans la zone à l'extérieur de chaque ligne (9) entre les rabats étanches (5) et la feuille arrière (2) et forme ainsi une première paire de terminaisons de jambe extérieures, et dont une seconde paire (12) de moyens d'élastification est localisée dans la zone entre les lignes (9) contre les rabats étanches (5) dans la zone de leur bord intérieur, pour former ainsi une seconde paire de terminaisons de jambe intérieures, la feuille arrière (2) étant perméable à la vapeur d'eau mais étanche aux liquides dans la zone centrale (Z) entre les limites (20), qui sont formées par la paire extérieure des moyens d'élastification (13), et perméable à l'air et à la vapeur d'eau dans les parties latérales (S) à l'extérieur de la zone centrale (Z) et des limites (20), **caractérisée en ce que** les rabats étanches (5) sont reliés de manière étanche à la feuille arrière (2) le long de chaque ligne (9) et forment chacun une barrière aux liquides, laquelle ferme la voie au liquide entre la feuille arrière (2) et les rabats étanches (5) dans les parties latérales (S) également perméables à l'air, et **en ce que** la perméabilité à la vapeur d'eau de la feuille arrière (2) est plus grande dans la zone centrale (Z) entre les limites (20) que dans les parties latérales (S) à l'extérieur de ces limites (20).

2. Couche jetable selon la revendication 1, **caractérisée en ce que** les moyens d'élastification (13) extérieurs, ou les terminaisons de jambe formées par ceux-ci, sont disposés entre les rabats étanches (5) et la feuille arrière (2) de telle manière qu'ils forment une autre barrière aux liquides, laquelle empêche une progression latérale du liquide dans la direction des parties latérales (S).

3. Couche jetable selon la revendication 1 ou 2, **caractérisée en ce que** la feuille arrière (2) est réalisée de telle manière qu'elle laisse passer la vapeur d'eau par le phénomène de chimisorption.

4. Couche jetable selon la revendication 3, **caractérisée en ce que** la propriété de la feuille arrière (2), consistant à laisser passer la vapeur d'eau par le phénomène de chimisorption, est limitée à la zone centrale (Z) entre les limites (20).

5. Couche jetable selon l'une des revendications précédentes, **caractérisée en ce que** la feuille arrière (2) présente des micropores pour laisser passer la vapeur d'eau.

6. Couche jetable selon la revendication 5, **caractérisée en ce que** les micropores sont limités à la zone centrale (Z) entre les limites (20).

7. Couche jetable selon la revendication 5 ou 6, **caractérisée en ce que** les micropores présentent un diamètre compris entre 0,2 et 10 µm.

8. Couche jetable selon l'une des revendications précédentes, **caractérisée en ce que** la feuille arrière (2) présente des macropores à l'extérieur de la zone centrale (Z) délimitée par les limites (20).

9. Couche jetable selon la revendication 8, **caractérisée en ce que** la taille de la surface de projection des macropores est comprise entre 0,10 et 5,0 mm².

10. Couche jetable selon la revendication 8 ou 9, **caractérisée en ce que** les rabats étanches (5) présentent des macropores à l'extérieur de la zone centrale (Z) délimitée par les limites (20).

11. Couche jetable selon la revendication 10, **caractérisé en ce que** les macropores des rabats étanches (5) présentent une surface de projection comprise entro 0,10 et 5,0 mm².

12. Couche jetable selon l'une des revendications précédentes, **caractérisée en ce que** la feuille arrière (2) présente une structure monocouche.

13. Couche jetable selon l'une des revendications précédentes, **caractérisée en ce que** la feuille arrière (2) comprend au moins par endroits un composite non-tissé/film de telle manière que les composants de film pénètrent dans la structure tridimensionnelle des composants de non-tissé.
